# EUROPÄISCHE PATENTSCHRIFT

(11) **EP 0 645 388 B1**
(45) Veröffentlichungstag und Bekanntmachung des Hinweises auf die Patenterteilung: **05.02.1997**
(21) Anmeldenummer: 94114550.0
(22) Anmeldetag: 15.09.1994
(51) Int. Cl.: C07D 471/04

(54) **Verfahren zur Herstellung von Imidazopyridinderivaten**
Process for the production of imidazopyridines
Procédé pour la préparation de derivés des imidazopyridines

(30) Priorität: 17.09.1993 CH 2815/93
(43) Veröffentlichungstag der Anmeldung: 29.03.1995
(73) Patentinhaber: LONZA AG, CH-3945 Gampel/Wallis (CH)
(72) Erfinder: Stucky, Gerhard, Dr., CH-3902 Brig-Glis (Kanton Wallis) (CH); Imwinkelried, René, Dr., CH-3902 Brig-Glis (Kanton Wallis) (CH)
(74) Vertreter: Weinhold, Peter, Dr.

(56) Entgegenhaltungen:
- EP-A- 0 130 461
- EP-A- 0 385 850
- EP-A- 0 510 813
- WO-A-93/23399
- US-A- 5 066 654
- US-A- 5 240 938

## Beschreibung

Die Erfindung betrifft ein neues Verfahren zur Herstellung von Imidazopyridinderivaten der allgemeinen Formel 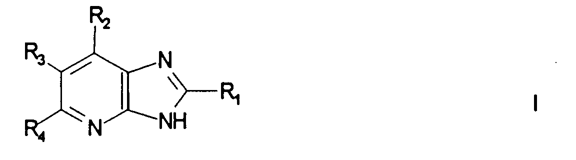
worin
- R₁: eine Alkyl-, Cycloalkyl-, Aryl- oder Aralkylgruppe bedeutet oder für einen heterocyclischen Rest steht,
- R₂ und R₄: gleich oder verschieden sind und Wasserstoff, eine Hydroxy-, eine Cyan-, Alkyl-, Cycloalkyl-, Aryl- oder Aralkylgruppe bedeuten oder für eine Alkanoyl- oder eine Alkoxycarbonylgruppe stehen und
- R₃: Wasserstoff, eine Alkyl-, Aryl- oder Aralkylgruppe oder ein Halogenatom bedeutet.

Diese Verbindungen finden Anwendung als Zwischenprodukte für die Herstellung von Angiotensin-II-Antagonisten (J. Med. Chem 1991, 34, 2919 - 2922).

In der genannten Literaturstelle wird beschrieben, dass die Imidazopyridine durch Reduktion von 2-Amino-3-nitropyridinen und anschliessende Kondensation mit einer entsprechenden aliphatischen Carbonsäure erhalten werden können.

Die Bereitstellung der Ausgangsprodukte der 2-Amino-3-nitropyridine gestaltet sich hingegen schwierig, da die Nitrierung der entsprechenden Aminopyridine nicht regioselektiv verläuft.

Die Aufgabe der Erfindung bestand folglich darin,ein Verfahren zu entwickeln, dass einen einfachen und grosstechnisch umsetzbaren Zugang zu den Imidazopyridinen der allgemeinen Formel I ermöglicht.

Die Aufgabe konnte gelöst werden mit dem Verfahren gemäss Anspruch 1.

Die für die einzelnen Reste R₁ bis R₇ und X verwendeten Begriffe haben nachfolgende Bedeutung:

Unter der Bezeichnung Alkylgruppe wird eine geradkettige oder verzweigte Alkylgruppe mit zweckmässig 1 bis 6 C-Atomen, vorzugsweise mit 1 bis 4 C-Atomen, verstanden.

Beispielhaft seien die Methyl-, Ethyl-, n-Propyl-, i-Propyl-, n-Butyl- oder die t-Butylgruppe genannt.

Unter der Bezeichnung Cycloalkylgruppe wird zweckmässig eine C₃-C₆-Cycloalkylgruppe, wie z. B. eine Cyclopropyl-, eine Cyclobutyl-, eine Cyclopentyl- oder eine Cyclohexylgruppe,verstanden.

Die Bezeichnung Aryl umfasst carbocyclische Aromaten, zweckmässig Phenyl oder Naphthyl,und
die Bezeichnung Aralkyl steht für eine arylsubstituierte Alkylgruppe, zweckmässig für eine phenylsubstituierte C₁-C₆-Alkylgruppe,insbesondere für Benzyl.

Unter einer Alkanoylgruppe wird zweckmässig eine (C₁-C₆)-Alkanoylgruppe, vorzugsweise Acetyl,verstanden.

Alkoxy bedeutet zweckmässig (C₁-C₆)-Alkoxy,vorzugsweise Methoxy oder Ethoxy.

Unter einem heterocyclischen Rest wird zweckmässig ein 5- oder 6-Ringheterocyclus mit Stickstoff und / oder Sauerstoff und / oder Schwefel als Heteroatom verstanden.
Ebenso zählen kondensierte Ringsysteme von Heterocyclen untereinander oder von Heterocyclen mit carbocyclischen Systemen unter den genannten Begriff. Beispielhaft für 5-Ring-Heterocyclen seien die Furane, die Thiophene, die Pyrrole, die Indole, die Pyrazole, die Imidazole, die Oxazole, Isoxazole, die Thiazole oder die Triazole genannt.

Als Beispiel für 6-Ring-Heterocyclen werden die Pyridine, die Chinoline, die Isochinoline, die Acridine, die Pyridazine, die Pyrimidine, die Pyrazine, die Phenazine, die Purine oder die Pteridine genannt.

Unter Halogen wird Fluor, Chlor, Brom oder Jod verstanden, bevorzugtes Halogen ist Chlor.

Die genannten Gruppen, insbesondere die cyclischen Reste, können jeweils einfach oder mehrfach substituiert sein. Geeignete Reste sind z. B. Halogen, Nitro, Amino, Alkylamino, Dialkylamino, Hydroxy, Alkoxy, Alkyl oder Alkanoyl. Für die Bedeutung dieser Reste sind die vorstehenden Erklärungen gültig.

In der ersten Stufe des erfindungsgemässen Verfahrens wird ein Nitril der allgemeinen Formel

R₁CN II

worin R₁ die genannte Bedeutung hat,mit einem Thiol der allgemeinen Formel

R₅SH III

worin R₅ Alkyl, Aryl oder Aralkyl bedeutet,in Gegenwart von einem Halogenwasserstoff zu einem Thioimidathydrohalogenid der allgemeinen Formel 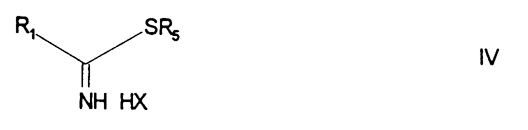
worin R₁ und R₅ die genannten Bedeutungen haben und X ein Halogenatom bedeutet, umgesetzt.
Diese erste Stufe wurde im wesentlichen von Bader et al. in J. Chem. Soc. 1950, 2780 beschrieben.
Zweckmässig wird als Nitril der allgemeinen Formel II Acetonitril, Propionitril, Butyronitril oder Valeronitril eingesetzt.

Besonders bevorzugtes Nitril der allgemeinen Formel II ist Propionitril.
Im weiteren wird bevorzugt Benzylmercaptan als Thiol der allgemeinen Formel III und Chlorwasserstoff als Halogenwasserstoff eingesetzt.

Grundsätzlich kann das eingesetzte Nitril als Lösungsmittel fungieren. In der Regel wird aber zusätzlich ein inertes Lösungsmittel wie z. B. Dioxan, Tetrahydrofuran, Ether, ein halogenierter
Kohlenwasserstoff wie z. B. Methylenchlorid oder ein aromatischer Kohlenwasserstoff wie Toluol eingesetzt.

Es hat sich gezeigt, dass die Umsetzung in der ersten Stufe zweckmässig bei Temperaturen zwischen 0°C und Raumtemperatur durchgeführt wird.
Die Reaktionszeit ist im wesentlichen abhängig von der Halogenwasserstoffmenge. Die besten Resultate werden im Bereich von 2 bis 3 Äquivalenten Halogenwasserstoff pro Äquivalent Thiol der allgemeinen Formel III erhalten.

Das resultierende Thioimidat der allgemeinen Formel IV kann auf fachmännische Weise isoliert werden, bevorzugt wird es aber direkt mit Aminoacetonitril zum Amidin der allgemeinen Formel 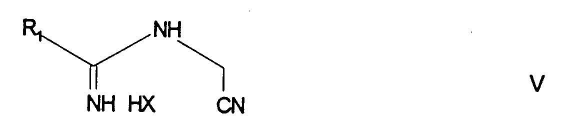
worin R₁ und X die genannten Bedeutungen hat, weiterumgesetzt.

Das Aminoacetonitril wird von Vorteil jeweils direkt vor der Reaktion aus einem entsprechenden Aminoacetonitrilsalz,wie z. B. dem Hydrochlorid oder dem Hydrosulfat,durch dessen Umsetzung mit einer Base, z. B. mit Ammoniak,freigesetzt. Üblicherweise wird die Reaktion im Lösungsmittel der Vorstufe durchgeführt.

Die Umsetzungstemperatur liegt zweckmässig zwischen 0°C und der Rückflusstemperatur des jeweiligen Lösungsmittels.

Das resultierende Amidin der allgemeinen Formel V kann nach beendeter Reaktion auf fachmännische Weise im Falle des Amidins der allgemeinen Formel V mit R₁ = Ethyl und X = Cl z. B. durch Filtration aus dem Reaktionsgemisch entnommen werden.

In der letzten Stufe wird das Amidin der allgemeinen Formel V in Gegenwart einer Base mit einer 1,3-Dicarbonylverbindung der allgemeinen Formel 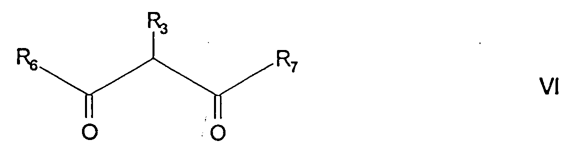 worin R₃ die genannte Bedeutung hat und R₆ und R₇ gleich oder verschieden sind und Wasserstoff, Alkyl, Aryl, Aralkyl, Alkoxy oder Alkoxycarbonyl bedeuten, zum Endprodukt cyclisiert.

Generell erfolgt der Ringschluß bei Temperaturen zwischen 0°C und Rückflußtemperatur, gegebenenfalls in Gegenwart eines inerten Lösungsmittels.

Geeignete 1,3-Dicarbonylverbindungen mit R₆ und R₇ = Alkyl sind die Alkandione wie z. B. das 2,4-Pentandion (Acetylaceton), das 3,5-Heptandion, das 4,6-Nonandion oder das 3-Methyl-2,4-pentandion (2-Methylacetylaceton) mit R₃ = Methyl.

Vertreter mit R₆ = Alkyl und R₇ = Alkoxy sind die Alkanoylessigsäureester wie z. B. der Acetessigsäuremethylester oder der Acetessigsäureethylester.

Zweckmässig werden auch die Malonester mit R₆ und R₇ = Alkoxy eingesetzt. Beispielhaft seien der Malonsäuremethylester und der Malonsäureethylester genannt.

Geeignete Verbindungen der allgemeinen Formel VI mit R₆ und R₇ = Wasserstoff sind der Malondialdehyd bzw. die 2-substituierten Malondialdehyde.

Weitere geeignete Vertreter der allgemeinen Formel VI mit R₆ und R₇ = Alkoxycarbonyl sind der 2,4-Dioxopentandisäuredimethylester oder der 2,4-Dioxopentandisäurediethylester mit R₆ und R₇ = Methoxycarbonyl und Ethoxycarbonyl.

Als Basen können anorganische oder organische Basen verwendet werden.

Zweckmässig wird als organische Base für die Cyclisierung ein Alkalialkoholat wie z. B. Na- oder K-ethanolat, Na- / K-methanolat oder K-t-butylat im entsprechenden Alkohol oder ein Trialkylamin wie z. B. Triethylamin verwendet.

Als anorganische Base werden zweckmässig Alkalihydroxide in einem niederen aliphatischen Alkohol wie z. B. NaOH oder KOH in Methanol oder Wasser aber auch Alkali- oder Erdalkalicarbonate oder -hydrogencarbonate eingesetzt.

Die Lösungsmittelwahl ist nicht sonderlich kritisch. Gute Resultate können mit niederen aliphatischen Alkoholen wie Methanol oder Ethanol aber auch mit aromatischen Kohlenwasserstoffen wie z. B. Toluol erhalten werden.

Die Umsetzung verläuft zweckmässig zwischen Raumtemperatur und Rückflusstemperatur des jeweiligen Lösungsmittels vorzugsweise zwischen 50°C und Rückflusstemperatur des Lösungsmittels.
Nach beendeter Reaktion kann das Imidazopyridin auf übliche Weise aus dem Reaktionsgemisch abgetrennt werden.

### Beispiel 1

a] Verfahren zur Herstellung des S-Benzylpropionthioamid · HCl
   Zu einer Lösung von 50.2 g (0.4 mol) Benzylmercaptan und 24.2 g (0.44 mol) Propionitril in 100 ml Dioxan wurde bei 10°C 43 g (1.2 mol; 3eq) HCl-Gas eingeleitet. Nach beendeter Einleitung (ca. 1 Stunde) wurde auf Raumtemperatur erwärmt. Nach 2.5 Stunden wurde ein Teil des Dioxans und überschüssiges HCl mit Vakuum abgezogen, wobei das Produkt ausfiel. Dieses wurde filtriert, mit wenig Ether gewaschen und am Vakuum getrocknet. Man erhält 82.1 g weisses Titelprodukt (95% Ausbeute).
   - ¹H-NMR: (CDCl₃, 300 MHz) δ: 1,4 (t, 3H);
   2,95 (q, 2H)
   4,25 (s, 2H)
   7,3 - 7,5 (m, 5H)
   12,2 (br. s, 1H)
   13,1 (br. s, 1H)
b] Verfahren zur Herstellung des (1-Iminopropylamino)-acetonitril · HCl
   Eine Suspension von 81.5 g (0.38 mol) des Produktes aus 1a in 300 ml Dioxan wurde auf ca. 10°C gekühlt und mit 23.8 g (0.42 mol) Aminoacetonitril (freigesetzt aus dem Aminoacetonitril-hydrochlorid mit Ammoniak) versetzt. Nach zwei Stunden bei dieser Temperatur wurde das Produkt filtriert, mit Ether gewaschen und am Vakuum getrocknet. Man erhielt 52.2 g weisses Titelprodukt (Ausbeute 93%).
   - Fp: 92 - 93°C:
   - ¹H-NMR: (DMSO, 400 MHz) δ: 1,2 (t, 3H)
   2,5 (q, 2H)
   4,6 (s, 2H)
   9,6 (s, 1H)
   10,0 (s, 1H)
   10,6 (s, 1H)
c] Verfahren zur Herstellung des (1-Iminopropylamino)-Acetonitril · HCl
   Zu einer Lösung von 12.4 g (0.1 mol) Benzylmercaptan und 6.1 g (0.11 mol) Propionitril in 25 ml Dioxan wurden bei 10°C 7.3 g (0.2 mol) HCl eingeleitet. Nach beendeter Einleitung wurde 17 Stunden bei Raumtemperatur gerührt. Das überschüssige HCl wurde am Vakuum abgezogen. Zur entstandenen Suspension wurde bei Raumtemperatur 6.16 g (0.11 mol) Aminoacetonitril (freigesetzt aus dem Hydrochlorid mit Ammoniak) zugetropft und zwei Stunden bei dieser Temperatur gerührt. Der entstandene Feststoff wurde filtriert, mit Ether gewaschen und am Vakuum getrocknet. Man erhielt 11 g weisses Titelprodukt (Ausbeute bezüglich Propionitril 75%)
d] Verfahren zur Herstellung von 2-Ethyl-5,7-dimethyl-3H-imidazo[4,5 - b]pyridin
   Zu einer Lösung von 35.4 g (0.24 mol) des Produktes aus 1c in 150 ml Ethanol wurde bei 0°C 150 ml einer 1.6M Natriumethanolatlösung in Ethanol (entspricht 0.24 mol NaOEt) zugetropft. Anschliessend wurden 240 g (2.4mol; 10 eq) Acetylaceton zugegeben, und das Reaktionsgemisch langsam auf 130°C erwärmt. Dabei wurde das Wasser und das Ethanol abdestilliert. Nach einer halben Stunde bei Rückflusstemperatur wurde auf Raumtemperatur abgekühlt, mit 500 ml Wasser und 500 ml Essigsäureethylester versetzt und die Phasen getrennt. Die organische Phase wurde mit MgSO₄ getrocknet und am Rotationsverdampfer eingeengt. Der erhaltene Rückstand wurde aus Essigsäureethylester umkristallisiert. Man erhielt 25.5 g (60%) leicht gelbliches Titelprodukt.
   - Fp: 148.8 - 150.4°C:
   - ¹H-NMR: (400 MHz in CD₃OD) δ: 1,4 (t, 3H)
   2,55 (s, 6H)
   2,9 (q, 2H)
   6,9 (s, 1H)

## Patentansprüche

1. Verfahren zur Herstellung von Imidazopyridinderivaten der allgemeinen Formel 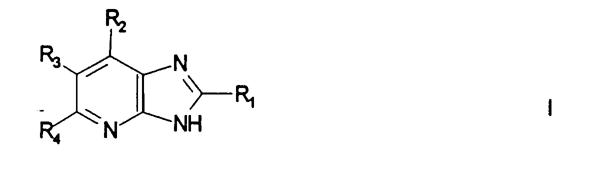 worin
R₁ eine Alkyl-, Cycloalkyl-, Aryl- oder Aralkylgruppe bedeutet oder für einen heterocyclischen Rest steht,
R₂ und R₄ gleich oder verschieden sind und Wasserstoff, eine Hydroxy-, eine Cyan-, Alkyl-, Cycloalkyl-, Aryl- oder Aralkylgruppe bedeuten oder für eine Alkanoyl- oder eine Alkoxycarbonylgruppe stehen und
R₃ Wasserstoff, eine Alkyl-, Aryl- oder Aralkylgruppe oder ein Halogenatom bedeutet, dadurch gekennzeichnet, dass man ein Nitril der
allgemeinen Formel
R₁CN II
worin R₁ die genannte Bedeutung hat,mit einem Thiol der allgemeinen Formel
R₅SH III
worin R₅ Alkyl, Aryl oder Aralkyl bedeutet, in Gegenwart von einem Halogenwasserstoff zu einem Thioimidathydrohalogenid der allgemeinen Formel 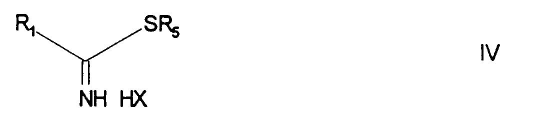 worin R₁ und R₅ die genannte Bedeutung haben und X ein Halogenatom bedeutet, umsetzt,dieses mit Aminoacetonitril zum Amidin der allgemeinen Formel 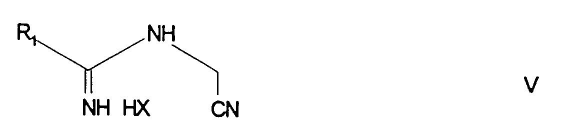 worin R₁ und X die genannten Bedeutungen haben,reagieren lässt und schliesslich in Gegenwart einer Base mit einer 1,3-Dicarbonylverbindung der allgemeinen Formel 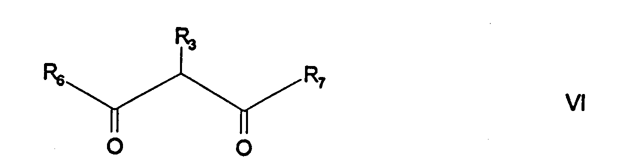 worin R₃ die genannte Bedeutung hat und R₆ und R₇ gleich oder verschieden sind und Wasserstoff oder Alkyl, Aryl, Aralkyl, Alkoxy oder Alkoxycarbonyl bedeuten,zum Endprodukt der allgemeinen Formel I ringschliesst.

2. Verfahren nach Patentanspruch 1, dadurch gekennzeichnet, dass das Thioimidathydrohalogenid der allgemeinen Formel IV nicht isoliert wird.

3. Verfahren nach Patentanspruch 1, dadurch gekennzeichnet, dass die Umsetzung zum Thioimidathydrohalogenid der allgemeinen Formel IV in Gegenwart von Chlorwasserstoff bei Reaktionstemperaturen zwischen 0°C und Raumtemperatur, gegebenenfalls unter Zusatz eines inerten Lösungsmittel, durchgeführt wird.

4. Verfahren nach mindestens einem der Patentansprüche 1 bis 3, dadurch gekennzeichnet, dass die Umsetzung zum Amidin der allgemeinen Formel V bei Temperaturen zwischen 0°C und Rückflusstemperatur des jeweiligen Lösungsmittels durchgeführt wird.

5. Verfahren nach mindestens einem der Patentansprüche 1 bis 4, dadurch gekennzeichnet, dass als Base für den Ringschluss eine anorganische oder organische Base verwendet wird.

6. Verfahren nach Patentanspruch 5, dadurch gekennzeichnet, dass als Base für den Ringschluss ein Alkalialkoholat oder ein Alkalihydroxid in einem niederen aliphatischen Alkohol eingesetzt wird.

7. Verfahren nach mindestens einem der Patentansprüche 1 bis 6, dadurch gekennzeichnet, dass der Ringschluss bei Temperaturen zwischen 0°C und Rückflusstemperatur, gegebenenfalls in Gegenwart eines inerten Lösungsmittels, erfolgt.

8. Verfahren nach mindestens einem der Patentansprüche 1 bis 7, dadurch gekennzeichnet, dass Aminoacetonitril aus einem Salz des Aminoacetonitrils mit Hilfe einer Base freigesetzt wird.

## Claims

1. A process for the preparation of imidazopyridine derivatives of general formula 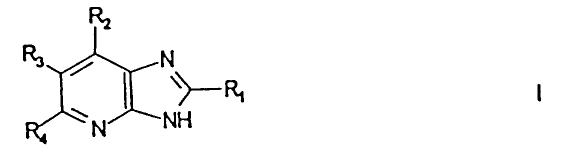 wherein
R₁ is an alkyl, cycloalkyl, aryl or aralkyl group or is a heterocyclic radical,
R₂ and R₄ are identical or different and are hydrogen, a hydroxy, cyano, alkyl, cycloalkyl, aryl or aralkyl group or are an alkanoyl or an alkoxy-carbonyl group, and
R₃ is hydrogen, an alkyl, aryl or aralkyl group or a halogen atom, characterized in that a nitrile of general formula
R₁CN II
wherein R₁ is as defined above, is reacted with a thiol of general formula
R₅SH III
wherein R₅ is alkyl, aryl or aralkyl, in the presence of a hydrogen halide, to give a thioimidate hydrohalide of general formula 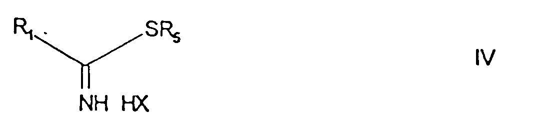 wherein R₁ and R₅ are as defined above and X is a halogen atom, which compound is reacted with aminoacetonitrile to give an amidine of general formula 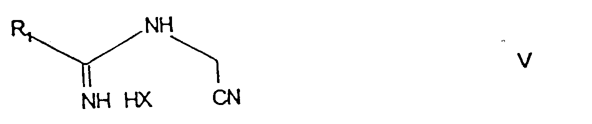 wherein R₁ and X are as defined above, and which is finally cyclized in the presence of a base with a 1,3-dicarbonyl compound of general formula 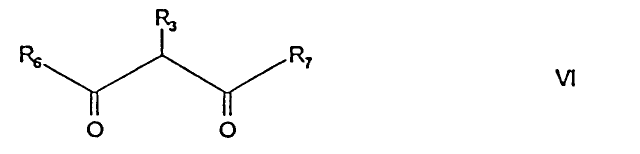 wherein R₃ is as defined above and R₆ and R₇ are identical or different and are hydrogen or alkyl, aryl, aralkyl, alkoxy or alkoxycarbonyl, to give the final product of general formula I.

2. The process according to claim 1, characterized in that the thioimidate hydrohalide of general formula IV is not isolated.

3. The process according to claim 1, characterized in that the reaction to give the thioimidate hydrohalide of general formula IV is carried out in the presence of hydrogen chloride and at a reaction temperature between 0 °C and room temperature, optionally with the addition of an inert solvent.

4. The process according to at least one of the claims 1 to 3, characterized in that the reaction to give the amidine of general formula V is carried out at a temperature between 0 °C and the reflux temperature of the respective solvent.

5. The process according to at least one of claims 1 to 4, characterized in that the base used for cyclization is an inorganic base or an organic base.

6. The process according to claim 5, characterized in that the base used for the cyclization is an alkali metal alkoxide, or an alkali metal hydroxide in a lower aliphatic alcohol.

7. The process according to at least one of the claims 1 to 6, characterized in that the cyclization is carried out at a temperature between 0 °C and reflux temperature, optionally in the presence of an inert solvent.

8. The process according to at least one of the claims 1 to 7, characterized in that the aminoacetonitrile is liberated from a salt of aminoacetonitrile by means of a base.

## Revendications

1. Procédé pour la préparation de dérivés d'imidazopyridine de la formule générale 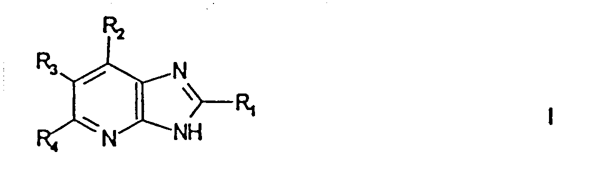 dans laquelle
R₁ signifie un groupe alkyle, cycloalkyle, aryle ou aralkyle ou un radical hétérocyclique,
R₂ et R₄ sont identiques ou différents et signifient l'hydrogène, un groupe hydroxy, un groupe cyano, alkyle, cycloalkyle, aryle ou aralkyle ou signifie un groupe alcanoyle ou un groupe alcoxycarbonyle, et
R₃ signifie l'hydrogène, un groupe alkyle, aryle ou aralkyle ou un atome d'halogène, caractérisé en ce que l'on met en réaction un nitrile de la formule générale
R₁CN II
dans laquelle R₁ a la signification citée avec un thiol de la formule générale
R₅SH III
dans laquelle R₅ signifie alkyle, aryle ou aralkyle, en présence d'un acide halohydrique pour obtenir un hydrocarbure halogéné de thioimidate de la formule générale 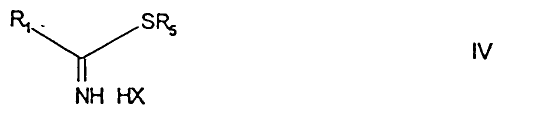 dans laquelle R₁ et R₅ ont la signification citée et X signifie un atome d'halogène, et on fait réagir celui-ci avec l'aminoacétonitrile pour donner l'amidine de la formule générale 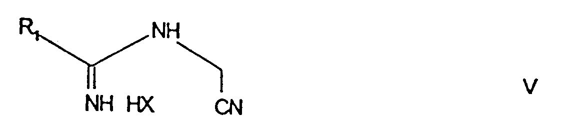 dans laquelle R₁ et X ont les significations citées et enfin on cyclise en présence d'une base avec un composé 1,3-dicarbonyle de la formule générale 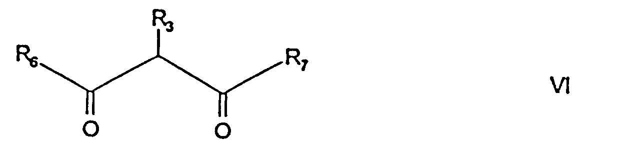 dans laquelle R₃ a la signification citée et R₆ et R₇ sont identiques ou différents et signifient l'hydrogène ou alkyle, aryle, aralkyle, alcoxy ou alcoxycarbonyle pour donner le produit final de la formule générale I.

2. Procédé selon la revendication 1, caractérisé en ce que l'hydrocarbure halogéné de thioimidate de la formule générale IV n'est pas isolé.

3. Procédé selon la revendication 1, caractérisé en ce que la réaction en hydrocarbure halogéné de thioimidate de la formule générale IV est conduite en présence de chlorure d'hydrogène à des températures réactionnelles entre 0°C et la température ambiante, éventuellement en ajoutant un solvant inerte.

4. Procédé selon au moins l'une des revendications 1 à 3, caractérisé en ce que la réaction en amidine de la formule générale V est conduite à des températures entre 0°C et la température de reflux du solvant éventuel.

5. Procédé selon au moins l'une des revendications 1 à 4, caractérisé en ce qu'en tant que base pour la cyclisation, on utilise une base minérale ou organique.

6. Procédé selon la revendication 5, caractérisé en ce qu'en tant que base pour la cyclisation, on met en oeuvre un alcoolat alcalin ou un hydroxyde alcalin dans un alcool aliphatique inférieur.

7. Procédé selon au moins l'une des revendications 1 à 6, caractérisé en ce que la cyclisation s'effectue à des températures entre 0°C et la température de reflux, éventuellement en présence d'un solvant inerte.

8. Procédé selon au moins l'une des revendications 1 à 7, caractérisé en ce que l'aminoacétonitrile est libéré à partir d'un sel d'aminoacétonitrile à l'aide d'une base.
